# EUROPEAN PATENT SPECIFICATION

(11) **EP 3 503 796 B1**
(45) Date of publication and mention of the grant of the patent: **04.06.2025**
(21) Application number: 17829580.4
(22) Date of filing: 22.08.2017
(51) Int. Cl.: A61B 5/024, A61B 5/0205, A61B 5/0245, A61B 5/01

(54) **HEART RATE MEASUREMENT METHOD AND DEVICE, AND WEARABLE APPARATUS**
HERZFREQUENZMESSVERFAHREN UND -VORRICHTUNG SOWIE WEARABLE-EINRICHTUNG
PROCÉDÉ ET DISPOSITIF DE MESURE DE FRÉQUENCE CARDIAQUE, ET APPAREIL PORTABLE

(30) Priority: 26.08.2016 CN 201610744267
(43) Date of publication of application: 03.07.2019
(73) Proprietor: BOE Technology Group Co., Ltd., Beijing 100015 (CN); Beijing BOE Multimedia Technology Co., Ltd., Beijing 100015 (CN)
(72) Inventor: LIN, Sen, Beijing 100176 (CN); WANG, Quanzhong, Beijing 100176 (CN)
(74) Representative: Isarpatent
(86) International application number: PCT/CN2017/098472
(87) International publication number: WO 2018/036474

(56) References cited:
- EP-A1- 3 026 523
- EP-A1- 3 231 361
- WO-A1-2016/096391
- WO-A1-2016/109918
- CN-A- 104 287 705
- CN-A- 105 266 789
- CN-A- 105 758 452
- US-A1- 2013 261 405
- US-A1- 2014 121 471
- US-A1- 2014 135 602
- US-A1- 2014 275 852
- US-A1- 2015 359 447
- US-A1- 2015 359 451
- US-A1- 2016 199 002
- US-A1- 2019 015 045

## Description

### CROSS-REFERENCE TO RELATED APPLICATION

The present application claims priority to Chinese Patent Application No. 201610744267.0 filed on August 26, 2016.

### TECHNICAL FIELD

The present disclosure is related generally to electronics technologies, and more specifically to a heart rate measurement method, a heart rate measurement device, and a wearable apparatus.

### BACKGROUND

With the improvement of quality of life, people are increasingly concerned about the health statuses of themselves, and correspondingly a variety of health care monitoring products are emerging on the market. As one of crucial physiological parameters, the heart rate of an individual has received a lot of attention, and has represented one of the most frequent health care monitoring targets.

On the other hand, smart electronics devices for terminal use, such as smart bracelets, smart watches, smart pendants, as well as other smart items convenient for wearing and carrying on, are also getting more and more favor on the market.

Currently on the market, there has been a trend to integrate the health care monitoring products and the smart electronics devices together, in order to realize an intelligent monitoring of the health status of a user, and to obtain real-time health data as well.

Heart rate monitoring has been a crucial part for monitoring of the health status of a user, and currently there have been a variety of different methods for measuring and monitoring heart rates of an individual. These methods include electrocardiography (ECG), whereby the bio-potential generated by electrical signals that control the expansion and contraction of heart chambers are measured, photoplethysmography (PPG), wherein a light-based technology is utilized to sense the rate of blood flow as controlled by the heart's pumping action, pulse oximetry, whereby a person's blood oxygen saturation is monitored by a pulse oximeter to thereby obtain a heart rate of the person, and an arterial pressure approach, whereby the heart rate is determined by measuring an arterial pressure of an individual.

The current photoplethysmography (PPG)-based heart rate measurement and detection device, because of its reliability in detection, small sizes of the circuit area and of the sensor electrode, no need for conductive media, and flexibility in deployment, has received a lot of attention, and has been widely employed in smart wristbands, smart watches, and other smart wearable apparatuses on the market.

The working principle of PPG detection of a heart rate detection device is as follows: a light-emitting sub-device first sheds a light beam of a certain wavelength to the blood vessels under the skin, then optical signals carrying information of the blood vessels and blood flow conditions are reflected or transmitted to a light-receiving sub-device for subsequent detection.

If a relatively large amount of blood is flowing in a blood vessel, the absorption of light by the blood and the blood vessel is also large, which in turn causes the light reflected or refracted by the blood vessel that reaches the light receiving sub-device has a relatively weak intensity. On the other hand, if a relatively small amount of blood is flowing in the blood vessel, the reflected or refracted light has a relatively strong intensity. By means of the corresponding relationship between heart rates and the intensity of the light received by the light receiving sub-device, the heart rate data can be obtained and determined.

There are a variety of movements of a human body, such as walking, jogging, and running, etc., and at a different movement state, the human body and the intelligent monitoring device have a different degree of fitness.

US 2015/359447 A1 discloses an electronic device wearable on a body and able to determine whether the electronic device is on the body.

US 2014/121471 A1 discloses an illumination source configured to illuminate the skin of the user.

US 2014/275852 A1 discloses a wearable fitness monitoring device including a motion sensor and a PPG sensor.

US 2013/261405 A1 discloses an apparatus and a method for easily and accurately measuring a biological signal by using a wristwatch-type measurement module.

US 2016/199002 A1 discloses a wearable device and a method for controlling the same by determining a distance between a user and the wearable device.

WO 2016/109918 A1 discloses a detection method for a wearable device to accurately determine whether the user is wearing the wearable device.

US 2015/359451 A1 discloses a heart rate meter and a method thereof by sensing circuit of a heart rate meter.

CN 105 758 452 A discloses an electronic device wearable on a body and a detection method which is able to determine whether the electronic device is on the body.

EP 3 026 523 A1 discloses a method and an apparatus for contacting skin with sensor equipment.

WO 2016/096391 discloses an apparatus comprising a sensor for measuring a physiological parameter of a subject.

### SUMMARY

In order to address the issues associated with the heart rate measurement and detection method that have been employed in current intelligent monitoring devices, the present disclosure provides a heart rate measurement method, a heart rate measurement device, and a wearable apparatus, which can be employed for accurate and real-time monitoring of the heart rates for a user.

In a first aspect, a method for monitoring heart rates of a user is disclosed herein. The user can be wearing a heart rate measurement device that is configured to detect biometric signals of the user, each at a first time interval. Each time when a biometric signal is to be detected, the method comprises the following steps:
detecting one biometric signal of the user and a distance signal reflecting and corresponding to a degree of fitness between the heart rate measurement device and skin of the user;
obtaining a heart rate value based on the one biometric signal, and determining whether the heart rate value is desirable based on the distance signal; and
keeping the heart rate value if the heart rate value is determined to be desirable, or discarding the heart rate value if otherwise.

Herein the biometric signals are at least one of photoplethysmography (PPG) signals, electrocardiography (ECG) signals, blood oxygen saturation signals, or arterial pressure signals. In some preferred embodiment, the biometric signals are PPG signals.

According to some embodiments of the method, in the step of keeping the heart rate value if the heart rate value is determined to be desirable, or discarding the heart rate value if otherwise, the discarding the heart rate value includes the following sub-steps:
starting timing; and
sending an action instruction to the heart rate measurement device to adjust the degree of fitness between the heart rate measurement device and the skin of the user if no heart rate value is determined as desirable within a time period, or stopping timing and discarding the heart rate value if otherwise.

Herein the biometric signals are at least one of photoplethysmography (PPG) signals, electrocardiography (ECG) signals, blood oxygen saturation signals, or arterial pressure signals. In some preferred embodiment, the biometric signals are PPG signals.

According to some embodiments of the method, in the step of keeping the heart rate value if the heart rate value is determined to be desirable, or discarding the heart rate value if otherwise, the discarding the heart rate value includes the following sub-steps:
starting timing; and
sending an action instruction to the heart rate measurement device to adjust the degree of fitness between the heart rate measurement device and the skin of the user if no heart rate value is determined as desirable within a time period, or stopping timing and resetting if otherwise.

In the method as described above, the action instruction can include a prompt instruction configured to prompt the user for manually adjusting the heart rate measurement device to be fit with the skin of the user. As such, the sub-step of sending an action instruction to the heart rate measurement device to adjust the degree of fitness between the heart rate measurement device and the skin of the user can include:
sending the prompt instruction to the heart rate measurement device.

In the method as described above, the action instruction can include an adjusting instruction configured to instruct the heart rate measurement device for automatically adjusting to be fit with the skin of the user. As such, the sub-step of sending an instruction to the heart rate measurement device to adjust the degree of fitness between the heart rate measurement device and the skin of the user can include:
sending the adjusting instruction to the heart rate measurement device.

According to the invention, prior to the step of detecting one biometric signal of the user and a distance signal reflecting and corresponding to a degree of fitness between the heart rate measurement device and skin of the user, the method further includes the following steps:
collecting physiological information of the user;
determining whether a body of the user is in a state of movement based on the physiological information; and
sending an action instruction to the heart rate measurement device to adjust the degree of fitness between the heart rate measurement device and the skin of the user if the body of the user is determined to be in the state of movement.

In the method as described above, the action instruction can include a prompt instruction configured to prompt the user for manually adjusting the heart rate measurement device to be fit with the skin of the user. As such, the sub-step of sending an action instruction to the heart rate measurement device to manually or automatically adjust the degree of fitness between the heart rate measurement device and the skin of the user can include:
sending the prompt instruction to the heart rate measurement device.

In the method as described above, the action instruction can include an adjusting instruction configured to instruct the heart rate measurement device for automatically adjusting to be fit with the skin of the user. As such, the sub-step sending an instruction to the heart rate measurement device to manually or automatically adjust the degree of fitness between the heart rate measurement device and the skin of the user can include:
sending the adjusting instruction to the heart rate measurement device.

In the method as described above, the distance signal can include at least one of a temperature signal, an optical pulse signal, or a second time interval signal.

According to some embodiments of the method where the distance signal includes a temperature signal. As such, the step of detecting one biometric signal of the user and a distance signal reflecting and corresponding to a degree of fitness between the heart rate measurement device and skin of the user can include:
detecting an instant temperature of the user by a temperature sensor;

In addition, the step of obtaining a heart rate value based on the one biometric signal and determining whether the heart rate value is desirable based on the distance signal can include the following sub-steps:
obtaining a change of temperature by comparing the instant temperature with a reference temperature; and
determining that the heart rate value is not desirable if the change of temperature is greater than a preset threshold, or determining that the heart rate value is desirable if otherwise.

According to some other embodiments of the method where the distance signal includes an optical pulse signal, the step of detecting one biometric signal of the user and a distance signal reflecting and corresponding to a degree of fitness between the heart rate measurement device and skin of the user can include:
detecting an instant optical pulse signal reflected from the skin of the user by a proximity sensor.

In addition, the step of obtaining a heart rate value based on the one biometric signal and determining whether the heart rate value is desirable based on the distance signal can include the following sub-steps:
obtaining a change of optical pulse signal by comparing the instant optical pulse signal with a reference optical pulse signal; and
determining that the heart rate value is not desirable if the change of optical pulse signal is greater than a preset threshold, or determining that the heart rate value is desirable if otherwise.

According to yet some other embodiments of the method where the distance signal includes a second time interval signal, the step of detecting one biometric signal of the user and a distance signal reflecting and corresponding to a degree of fitness between the heart rate measurement device and skin of the user can include:
detecting an instant second time interval signal of an infrared light reflected from the skin of the user by a distance sensor;

In addition, the step of obtaining a heart rate value based on the one biometric signal and determining whether the heart rate value is desirable based on the distance signal can include the following sub-steps:
comparing the instant second time interval signal with a preset threshold; and
determining that the heart rate value is not desirable if the instant second time interval signal is greater than the preset threshold, or determining that the heart rate value is desirable if otherwise.

In a second aspect, the disclosure further provides a heart rate measurement device.

The heart rate measurement device includes a heart rate detecting sensor, a fitness detecting sensor, and a processor. The heart rate detecting sensor is coupled to the processor, and is configured to detect, and to transmit to the processor, one biometric signal of a user at a first time interval; the fitness detecting sensor is coupled to the processor, and is configured to detect, and to transmit to the processor, a distance signal corresponding to the one biometric signal, wherein the distance signal reflects and corresponds to a degree of fitness between the heart rate measurement device and the skin of the user; and the processor is configured to obtain a heart rate value based on the one biometric signal, and to determine whether the heart rate value is desirable based on the distance signal.

The heart rate measurement device can further include a memory, which is coupled to the processor and is configured to store the heart rate value if the heart rate value is determined by the processor to be desirable.

According to some embodiments, the heart rate measurement device further includes an action portion coupled to the processor, and as such, the processor is further configured to start timing if determining that the heart rate value is not desirable, and to send an action instruction to the action portion if no heart rate value is determined as desirable within a time period, or to stop timing and reset if otherwise; and the action portion is configured, upon receiving the action instruction from the processor, to act on the heart rate measurement device to adjust the degree of fitness between the heart rate measurement device and the skin of the user.

In the heart rate measurement device as described above, the action portion can include a prompting portion, and accordingly the action instruction comprises a prompt instruction. The prompting portion is configured, upon receiving the prompt instruction from the processor, to prompt the user for manually adjusting the heart rate measurement device to be fit with the skin of the user.

In the heart rate measurement device as described above, the action portion can include an adjusting portion, and accordingly the action instruction comprises an adjusting instruction. The adjusting portion is configured, upon receiving the adjusting instruction from the processor, to automatically adjust the heart rate measurement device to be fit with the skin of the user.

According to some embodiments of the disclosure, the heart rate measurement device further includes a physiological information collecting portion and an action portion, each coupled to the processor.

The physiological information collecting portion is configured to collect physiological information of the user, and to send the physiological information to the processor.

Accordingly, the processor is further configured to determine whether a body of the user is in a state of movement based on the physiological information, and if so, to start timing and to send an action instruction to the action portion, or if otherwise, to stop timing and reset.

Furthermore, the action portion is configured, upon receiving the action instruction from the processor, to act on the heart rate measurement device to adjust the degree of fitness between the heart rate measurement device and the skin of the user.

In the heart rate measurement device as described above, the action portion can comprise a prompting portion, and accordingly the action instruction comprises a prompt instruction. As such, the prompting portion is configured, upon receiving the prompt instruction from the processor, to prompt the user for manually adjusting the heart rate measurement device to be fit with the skin of the user.

In the heart rate measurement device as described above, the action portion can include an adjusting portion, and accordingly the action instruction comprises an adjusting instruction. As such, the adjusting portion is configured, upon receiving the adjusting instruction from the processor, to automatically adjust the heart rate measurement device to be fit with the skin of the user.

In the heart rate measurement device, the prompting portion can include at least one of a display sub-portion, a vibration sub-portion, an audio sub-portion, or a light-emitting sub-portion.

In some embodiments of the heart rate measurement device, the prompting portion includes a display sub-portion, configured to display the heart rate value.

According to some other embodiments of the disclosure, the adjusting portion includes a wearing sub-portion and a fastening sub-portion. The wearing sub-portion is configured to attach the heart rate measurement device on a part of the human body of the user; and the fastening sub-portion is disposed on the wearing sub-portion and is configured to fasten or loosen the wearing sub-portion to thereby adjust a degree of fitness between the heart rate measurement device and the skin of the user based on the adjusting instruction from the processor.

In the heart rate measurement device as described above, the fitness detecting sensor can include at least one of a temperature sensor, a proximity sensor, or a distance sensor.

In the embodiments of the heart rate measurement device where the fitness detecting sensor includes a temperature sensor, the temperature sensor is configured to detect an instant temperature of the user, and the processor is further configured to obtain a change of temperature by comparing the instant temperature with a reference temperature, and to determine that the heart rate value is not desirable if the change of temperature is greater than a preset threshold, or that the heart rate value is desirable if otherwise.

In the embodiments of the heart rate measurement device where the fitness detecting sensor includes a proximity sensor, the proximity sensor is configured to detect an instant optical pulse signal reflected from the skin of the user, and the processor is further configured to obtain a change of optical pulse signal by comparing the instant optical pulse signal with a reference optical pulse signal, and to determine that the heart rate value is not desirable if the change of optical pulse signal is greater than a preset threshold, or that the heart rate value is desirable if otherwise.

In the embodiments of the heart rate measurement device where the fitness detecting sensor includes a distance sensor, the distance sensor is configured to detect an instant second time interval signal of an infrared light reflected from the skin of the user, and the processor is further configured to compare the instant second time interval signal with a preset threshold, and to determine that the heart rate value is not desirable if the instant second time interval signal is greater than the preset threshold, or that the heart rate value is desirable if otherwise.

In a third aspect, a wearable apparatus is disclosed, which includes a heart rate measurement device according to any of the embodiments as described above. The heart rate measurement device is disposed on a side of a user in contact with the skin of the user.

In the heart rate measurement device and method disclosed herein, a fitness detecting sensor is arranged in the heart rate measurement device, and is configured to detect a distance between the heart rate measurement device, which reflects a degree of fitness between the heart rate measurement device and skin of a user. The distance is then transmitted from the fitness detecting sensor to the processor,

The processor is configured to determine if the heart rate measurement device is fit with skin of a user, and based on the above determination result, to determine if the heart rate value obtained based on a PPG signal detected by the heart rate detecting sensor is desirable. The heart rate value can be stored in the memory if so, and can be removed if otherwise.

As such, inaccurate heart rate values can be removed, and the heart rate values that have been stored in the memory are each accurate heart rate value, thereby improving the heart rate detecting result.

### BRIEF DESCRIPTION OF THE DRAWINGS

To more clearly illustrate some of the embodiments, the following is a brief description of the drawings. The drawings in the following descriptions are only illustrative of some embodiment. For those of ordinary skill in the art, other drawings of other embodiments can become apparent based on these drawings.
FIG. 1 illustrates a schematic diagram of a heart rate measurement device according to a first embodiment of the present disclosure;
FIG. 2 illustrates a schematic diagram of a heart rate measurement device according to a second embodiment of the present disclosure;
FIG. 3 illustrates a schematic diagram of a heart rate measurement device according to a third embodiment of the present disclosure;
FIG. 4 illustrates a schematic diagram of a heart rate measurement device according to a fourth embodiment of the present disclosure;
FIG. 5 illustrates a schematic diagram of a heart rate measurement device according to a fifth embodiment of the present disclosure;
FIG. 6A illustrates a schematic diagram of a heart rate measurement device according to a sixth embodiment of the present disclosure;
FIG. 6B illustrates a schematic diagram of an adjusting portion in the heart rate measurement device when it is at a fastening state;
FIG. 6C illustrates a schematic diagram of an adjusting portion in the heart rate measurement device when it is at a loosening state;
FIG. 6D illustrates a schematic diagram of an adjusting portion in the heart rate measurement device according to some embodiments of the present disclosure;
FIG. 7 illustrates a schematic diagram of connections among various components of a heart rate measurement device according to a seventh embodiment of the present disclosure;
FIG. 8 shows a flow chart of a method for measuring a heart rate of a user by means of a heart rate measurement device according to some embodiments of the present disclosure;
FIG. 9 shows a flow chart of a method for measuring a heart rate of a user by means of a heart rate measurement device according to some other embodiments of the present disclosure.

### DETAILED DESCRIPTION

In the following, with reference to the drawings of various embodiments disclosed herein, the technical solutions of the embodiments of the disclosure will be described in a clear and fully understandable way.

It is obvious that the described embodiments are merely a portion but not all of the embodiments of the disclosure. Based on the described embodiments of the disclosure, those ordinarily skilled in the art can obtain other embodiment(s), which come(s) within the scope sought for protection by the disclosure.

In a first aspect, the present disclosure provides a heart rate measurement device. The heart rate measurement device can be an independent device worn by a user and specifically configured for measuring heart rates of a user, but can also be an functional module in a wearable apparatus worn by an individual that has a functionality of measuring heart rates for the user.

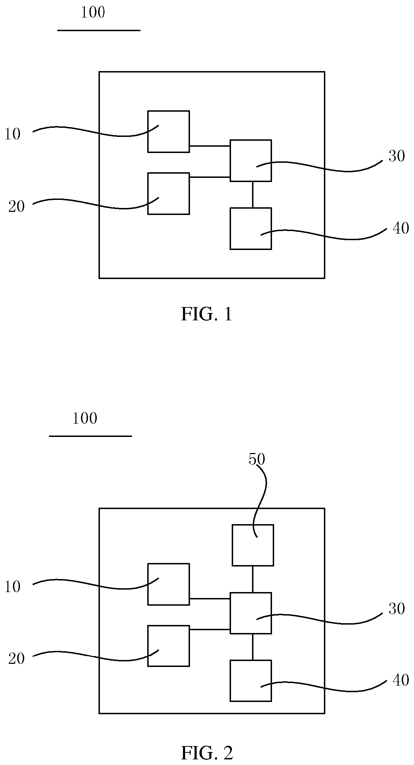

FIG. 1 illustrates a schematic diagram of a heart rate measurement device according to a first embodiment of the present disclosure. As shown in FIG. 1, the heart rate measurement device 100 comprises a heart rate detecting sensor 10, a fitness detecting sensor 20, a processor 30, and a memory 40.

The heart rate detecting sensor 10 is configured to detect, and to transmit to the processor 10, a photoplethysmography (PPG) signal in a body of a user.

The fitness detecting sensor 20 is configured to detect, and to transmit to the processor 10, a distance signal comprising a value of distance between the heart rate measurement device 100 and a human skin of the user. Herein the value of distance between the heart rate measurement device 100 and the human skin of the user is configured to reflect, or correspond to, a degree of fitness between the heart rate measurement device 100 and the human skin of the user.

The processor 30 is configured to obtain a heart rate value based on the PPG signal transmitted from the heart rate detecting sensor 10, to determine whether the heart rate value is desirable based on the distance value transmitted from the fitness detecting sensor 20, and to store the heart rate value in the memory 40 if so, or to remove the heart rate value if otherwise.

The following are noted.

First, there is no limitation to the specific types or structures of the heart rate measurement device 100, as long as the heart rate measurement device 100 touches the human skin after wearing.

Second, in this specific embodiment as described above, the heart rate detecting sensor 10 utilizes a PPG-based heart rate determining method for the determination of a heart rate of a user. The heart rate detecting sensor 10 can also utilize other methods, including an ECG-based, a pulse oximetry-based, or an arterial pressure-based heart rate determining method for the determination of a user's heart rate.

Third, there is no limitation to the specific types or structures of the fitness detecting sensor 20, as long as the fitness detecting sensor 20 can transmit to the processor 30 a signal corresponding to a degree of fitness between the heart rate measurement device 100 and the human skin.

Fourth, it is further noted that in addition to the PPG-based method that is utilized by the heart rate detecting sensor 10 in the heart rate measurement device 100 for the determination of a user's heart rate, other methods, including an ECG-based heart rate determining method, a pulse oximetry-based heart rate determining method, or an arterial pressure-based heart rate determining method, can also be utilized by the heart rate detecting sensor 10 in the heart rate measurement device 100 for determining a heart rate of the user. For convenience and simplicity, only the PPG-based method is described in detail as an illustrating example in the disclosure.

Herein the processor 30 can determine whether or not the heart rate measuring device 100 is fit with the human skin of the user based on the distance value transmitted from the fitness detecting sensor 20.

If the heart rate measuring device 100 is determined not to be fit with the human skin based on the distance signal, the heart rate values that are obtained after calculation over the PPG signal transmitted from the heart rate detecting sensor 10 are determined as not desirable.

If, on the other hand, the heart rate measuring device 100 is determined to be fit with the human skin, the heart rate values that are obtained after calculation over the PPG signal transmitted from the heart rate detecting sensor 10 are determined as desirable.

The processor 30 can be arranged on a printed circuit board (PCB), and can be a master chip. Herein the PCB can be arranged on the heart rate measurement device 100. Other embodiments are also possible.

The memory 40 can be integrated into the processor 30, but can also function as a component of the heart rate measurement device 100. Alternatively, the memory 40 can be realized by externally inserting a memory card into a slot that has been arranged. Other embodiments are possible, and there are no limitations herein.

In the above embodiment of the heart rate measurement device 100, the heart rate detecting sensor 10, the fitness detecting sensor 20, the processor 30, and the memory 40 all need a power supply to work functionally. As such, the heart rate measuring device 100 can further include a power supply (not shown), which can be arranged on a PCB, or may be placed elsewhere in other places according to the structure of the heart rate measuring device 100, as long as the power supply can supply power to the various components in the heart rate measuring device 100.

Specifically, if the power supply is a battery, it may be disposed inside the heart rate measuring device 100, or inside another electronics apparatus (such as a wearable apparatus) that also comprises the heart rate measurement device 100. If the power supply is a solar cell, it may be disposed on an outer surface of the heart rate measuring device 100 so as to facilitate providing the power supply to the heart rate measurement device 100 at any time. It is also possible to provide a charging interface on the heart rate measuring device 100 through which the battery can be charged.

The embodiment as described above does not impose limitations to the specific configuration of the heart rate detecting sensor 10, the fitness detecting sensor 20, the processor 30, and the memory 40, which can be configured based on the structure of the heart rate measurement device 100.

In the above embodiment of the heart rate measurement device 100, a fitness detecting sensor 20 is arranged in the heart rate measurement device 100, and is configured to detect a distance signal that reflects and corresponds to a degree of fitness between the heart rate measurement device 100 and skin of a user. The distance signal is then transmitted to the processor 30.

By determining whether or not the heart rate measuring device 100 is fit with the human skin of the user, the processor 30 can determine whether or not heart rate values obtained by calculating the photoplethysmography (PPG) signal of the blood detected by the heart rate detecting sensor 10 is desirable.

If the heart rate measuring device 100 is not fit with the human skin of the user, the heart rate values that are obtained after calculation are determined as not reliable or desirable, and are then removed. If the heart rate measuring device 100 is fit with the human skin of the user, the heart rate values that are obtained after calculation are determined as reliable and desirable, and are then stored in the memory 40..

As such, the heart rate values that are determined to be inaccurate can be removed, and the heart rate values stored in the memory 40 can only be accurate heart rate values, thereby improving the accuracy of the heart rate detection results.

FIG. 2 illustrates a schematic diagram of connections among various components of a heart rate measurement device according to a second embodiment of the present disclosure.

As shown in FIG. 2, the heart rate measurement device 100 further comprises a prompting portion 50, configured to prompt to the user that the heart rate measurement device 100 needs to be adjusted to be fit with the skin of the user.

The processor 30 is further configured to start timing if determining that the heart rate value is not desirable, and to send a prompt instruction to the prompting portion 50 if determining that none of the heart rate values collected by the heart rate detecting sensor 10 within a time period is desirable, or to stop timing and reset if determining that there is at least one desirable heart rate value within the time period. Herein, resetting refers to setting the processor to its original status (i.e. before determining whether there is at least one desirable heart rate value within a time period).

The prompting portion 50 is configured to send a prompt to the user based on the prompt instruction sent by the processor 30, which is purported to prompt the user that the heart rate measurement device 100 needs to be adjusted so as to be fit with the human skin.

Herein, the prompting portion 50 can comprise at least one of a display sub-portion, a vibration sub-portion, an audio sub-portion, or a light-emitting sub-portion, so as to meet the different requirements under a variety of different situations.

For example, if a display sub-portion is utilized in the prompting portion 50, the prompt can be displayed on a screen, and optionally, the heart rate values can be configured to be displayed on the screen as well.

Similarly, if the prompting portion 50 comprises a vibration sub-portion, an audio sub-portion, or a light-emitting sub-portion, the prompt can be a vibration cue, a sound cue, or an optical cue, that is presented to the user.

Upon receiving the prompt from the prompting portion 50, the user can manually adjust the heart rate measurement device 100 to be fit with the skin.

Herein by configuring a prompting portion 50 in the heart rate measurement device 100, if none of the heart rate values is determined as desirable within a time period, the prompting portion 50 sends a prompt to the user based on the prompt instruction sent from the processor 30, so that the user can adjust the heart rate measurement device 100 to be fit with the skin to thereby obtain the heart rate values accurately, realizing a real-time monitoring of the heart rate of the user.

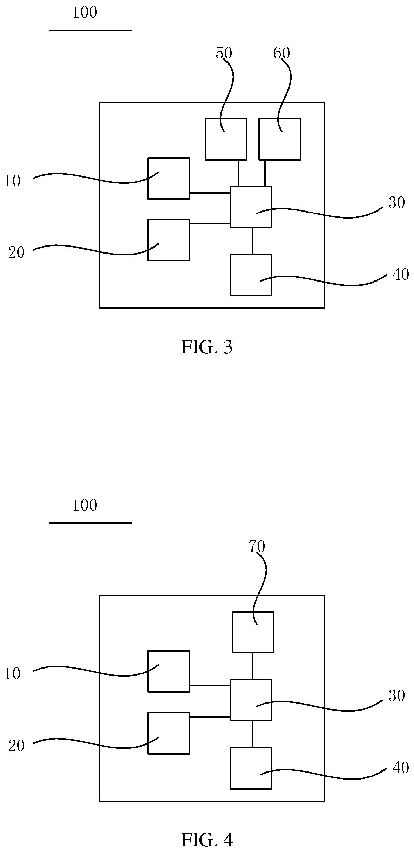

FIG. 3 illustrates a schematic diagram of a heart rate measurement device according to a third embodiment of the present disclosure. As shown in FIG. 3, the heart rate measurement device 100 further comprises a physiological information collecting portion 60.

The physiological information collecting portion 60 is configured to collect physiological information of the user in a real-time manner, and to send the physiological information to the processor 30. Correspondingly, the processor 30 is further configured to determine whether a body of the user is in a state of movement based on the physiological information collected from the physiological information collecting portion 60, and to send a prompt instruction to the prompting portion 50 if so.

Herein the physiological information that is collected by the physiological information collecting portion 60 can comprise a variety of information, such as pulse rate, body temperature, and heart beats, etc. The state of movement is referred to as a state of the user when the user is moving, and if the user is not moving, the user is not in the state of movement.

In an illustrating example where physiological information comprises body temperature, the physiological information collecting portion 60 comprises a thermometer. Because an individual typically has a different body temperature under a different state of movement, thus the processor 30 can perform an analysis over the body temperatures collected and sent by the physiological information collecting portion 60 to thereby determine the state of movement at the instant time.

Herein, by configuring a physiological information collecting portion 60 in the heart rate measurement device 100, the heart rate measurement device 100 can intelligently recognize a state of movement of the user, and can determine whether to send a prompt instruction to the prompting portion 50 based on the state of movement of the user so as to prompt the user to adjust the heart rate measurement device 100 to be fit with the skin to thereby obtain the heart rate values accurately.

FIG. 4 illustrates a schematic diagram of a heart rate measurement device according to a fourth embodiment of the present disclosure. As shown in FIG. 4, the heart rate measurement device 100 further comprises an adjusting portion 70, which is configured to adjust the heart rate measurement device 100 to be fit with the skin of the user based on an adjusting instruction from the processor 30.

The processor 30 is further configured to start timing if determining that the heart rate value is not desirable, and is configured to send the adjusting instruction to the adjusting portion 70 to decrease a distance between the heart rate measurement device 100 and the skin of the user if determining that none of the heart rate values is desirable within a time period, or to stop timing and reset if determining that there is at least one desirable heart rate value within the time period.

Herein there are no limitations to the specific structure of the adjusting portion 70, as long as the adjusting portion 70 can automatically adjust the heart rate measurement device 100 to be fit with the skin of the user.

Herein, by configuring an adjusting portion 70 in the heart rate measurement device 100, the adjusting portion 70 can automatically adjust the heart rate measurement device 100 to be fit with the skin of the user based on the adjusting instruction from the processor 30 so as to obtain accurate heart rate values to thereby realize a real-time monitoring of the heart rates of the user.

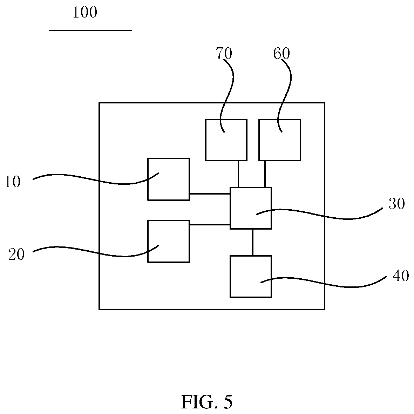

On top of the fourth embodiment of the heart rate measurement device 100 as shown in FIG. 4, the heart rate measurement device 100 further comprises a physiological information collecting portion 60, as illustrated in the fifth embodiment of the heart rate measurement device 100 shown in FIG. 5.

The physiological information collecting portion 60 is configured to collect physiological information of the user in a real-time manner, and to send the physiological information to the processor 30. The processor 30 is further configured to determine whether a human body of the user is in a state of movement based on the physiological information collected by the physiological information collecting portion 60, and if so, to send an adjusting instruction to the adjusting portion 70 so as to decrease a distance between the heart rate measurement device 100 and the skin of the user.

Herein, by configuring a physiological information collecting portion 60 in the heart rate measurement device 100, the heart rate measurement device 100 can intelligently recognize a state of movement of the user, and can determine whether to send an adjusting instruction to the adjusting portion 70 based on the state of movement of the user so as to automatically adjust a distance, and to thereby ensure a fitness, between the heart rate measurement device 100 and the skin of the user, thereby obtaining the heart rate values accurately.

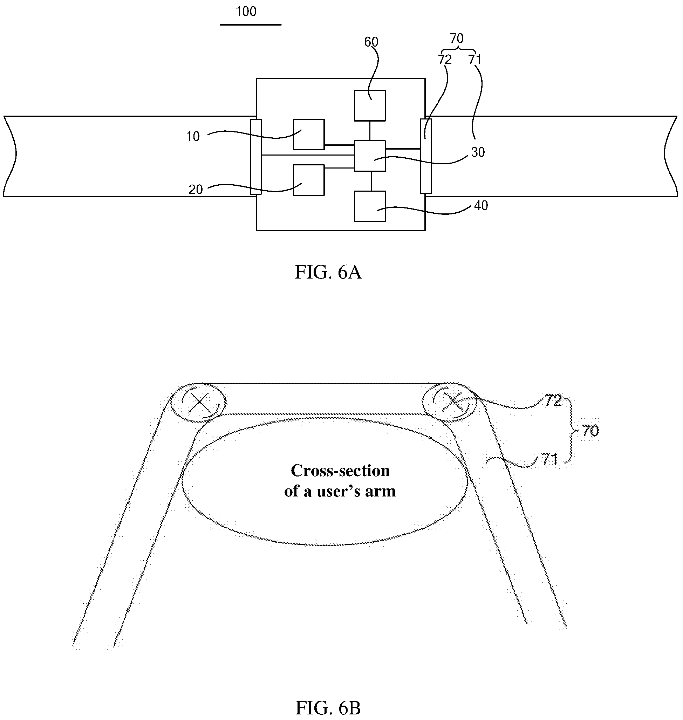

FIGS. 6A-6D illustrates several embodiments of the adjusting portion 70 of the heart rate measurement device 100. As shown in these figures, the adjusting portion 70 comprises a wearing sub-portion 71, and a fastening sub-portion 72 disposed on the wearing sub-portion 71.

The wearing sub-portion 71 is configured to attach the heart rate measurement device 100 on a part of the human body of the user. For example, the wearing sub-portion 71 can be a band, that is worn on a wrist, an ankle, an arm, a leg etc. of the user. The wearing sub-portion 71 can also be a patch with the heart rate measurement device 100, that is attached on a piece of skin on certain part of the body, such as a chest of the user.

The fastening sub-portion 72 is configured to fasten or loosen the wearing sub-portion 71 to thereby adjust a degree of fitness between the heart rate measurement device 100 and the skin of the user based on the adjusting instruction from the processor 30.

Herein there are no limitations to the specific structure and/or the number of the fastening sub-portion 72. There can be one or multiple fastening sub-portions 72 in different embodiment of the heart rate measurement device 100. For example, the embodiments of the heart rate measurement device 100 as illustrated in FIGS. 6A-6C have two fastening sub-portions 72.

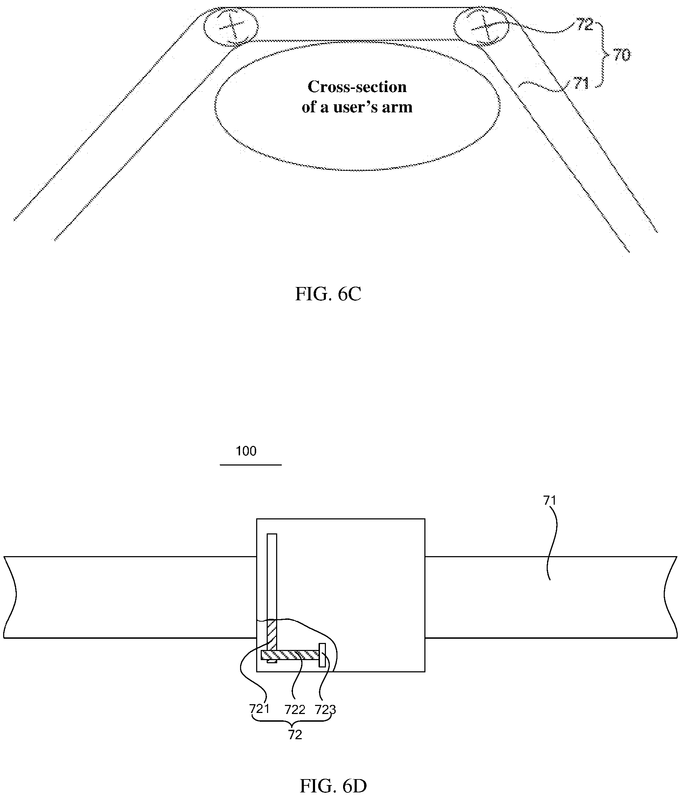

As illustrated in FIG. 6D, in some embodiments of the present disclosure, the fastening sub-portion 72 can comprise a worm 721, a worm wheel 722, and a motor 723. The worm 721 is connected to the wearing sub-portion 71, and the worm wheel 722 is connected to the worm 721. The motor 723 is configured to rotate the worm wheel 722.

Specifically, the processor 30 sends the adjusting instruction to the fastening sub-portion 72 to thereby start the motor 723, which in turn drives the worm wheel 722 to rotate. Rotation of the worm wheel 722 in turn drives the worm 721 to rotate so as to realize a fastening or loosening of the wearing sub-portion 71. The fastening state and the loosening state of the wearing sub-portion 71 are respectively shown in FIG. 6B and FIG. 6C.

Herein, by means of the fastening sub-portion 72, which is further configured to fasten or loosen the wearing sub-portion 71 based on the adjusting instruction from the processor 30, thereby realizing an intelligent adjustment of the degree of fitness between the heart rate measurement device 100 and the skin of the user.

In any of the embodiments as described above, the heart rate measurement device 100 can be a smart wristband, a smart footband, a smart neckband, or a smart wearable apparatus.

According to some embodiments, the fitness detecting sensor 20 comprises a temperature sensor, configured to detect a temperature which reflects and corresponds to a distance between the heart rate measurement device 100 and the skin of the user. The temperature sensor is disposed on a side of the heart rate measurement device 100 that is in contact with the skin of the user.

The temperature sensor can be a contact-type temperature detecting sub-device, which can be, for example, a thermistor. The thermistor utilizes the following principle: when a surface of the thermistor is in contact with a spot of a surface of an object with different temperatures, a resistance of the thermistor changes rapidly. After reading a real-time resistance of the thermistor through a circuit, the real-time resistance of the thermistor is then converted to thereby obtain an instant temperature of the spot of the surface of the object contacted by the thermistor.

The instant temperature is then transmitted to the processor 30, and the processor 30 obtains a temperature change by comparing the instant temperature with a reference temperature, wherein the reference temperature is a temperature of the body of the user when the heart rate measuring device 100 is closely fit with the human skin.

Because the body temperature typically does not have a great change even during an intense activity, thus if the temperature change obtained by the processor 30 is greater than a preset threshold, the processor 30 can determine that the heart rate measuring device 100 is not fit with the skin of the human body at the instant time.

Herein, by configuring a temperature sensor as the fitness detecting sensor 20 in the heart rate measurement device 100, on the one hand, the fitness detecting sensor 20 can be used to detect a distance between the heart rate measurement device 100 and the skin of the user, and on the other hand, the fitness detecting sensor 20 can be used to determine a health state of the user using the body temperature as one parameter for health of the user.

It is noted that the fitness detecting sensor 20 in the heart rate measurement device 100 can be other types of sensor, such as a proximity sensor or a distance sensor.

Specifically, a proximity sensor determines a distance between the proximity sensor and the skin of a user by transmitting an optical pulse of extremely short wavelength and measuring the intensity of the optical pulse that is reflected back. Thus after being reflected by the skin of the user, an instant optical pulse signal is received by a receiver in the proximity sensor, and the proximity sensor further sends the instant optical pulse signal to the processor 30.

The processor 30 compares the instant optical pulse signal with a reference optical pulse signal to thereby obtain a change of the optical pulse signal. The reference optical pulse signal is an optical pulse signal received by the receiver if the heart rate measurement device 100 is closely fit with the skin of the user.

Because of a relatively short wavelength, the optical pulse signal is unable to transmit to a relatively long distance. Thus if the heart rate measurement device 100 is not fit with, or is relatively far away from, the skin of a user, the receiver in the fitness detecting sensor 20 can only receive a relatively weak, or no, optical pulse signal.

As such, if the change of the optical pulse signal is greater than a preset threshold, the processor 30 can determine that the heart rate measurement device 100 and the skin of the user have a relatively low degree of fitness.

Specifically, a distance sensor transmits an infrared light and measures a distance between the distance sensor and the skin of a user by measuring a time interval for the infrared light to travel after emission until reflection back from an object. By measuring the time interval, a distance between the distance sensor and the skin of a user can be calculated.

After reflection by the skin of the user, the infrared light can be received by a receiver in the distance sensor. Then the distance sensor calculates a time interval between an emission of the infrared light and reception of the infrared light, and further sends the time interval to the processor 30.

The more distant the infrared light is from the skin of a user, the longer time interval for the infrared light to be reflected back after emission. As such, the processor 30 compares an instant time interval with a preset threshold, and if the instant time interval is greater than a preset threshold, the processor 30 can determine that there is a relatively low degree of fitness between the heart rate measurement device 100 and the skin of the user.

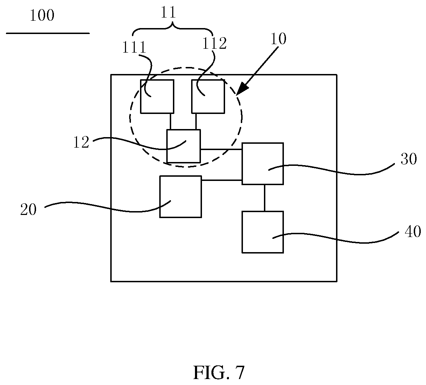

FIG. 7 illustrates a schematic diagram of a heart rate measurement device according to a seventh embodiment of the present disclosure. As shown in FIG. 7, the heart rate detecting sensor 10 of the heart rate measurement device 100 comprises a light-emitting circuit 11 and a light-receiving circuit 12.

The light-emitting circuit 11 comprises a first light-emitting sub-circuit 111 and a second light-emitting sub-circuit 112, wherein the first light-emitting sub-circuit 111 is configured to emit a green light, and the second light-emitting sub-circuit 112 is configured to emit a red light.

The light-emitting circuit 11 and the light-receiving circuit 12 can be disposed on a same side, or on opposing sides, of the heart rate measurement device 100. If the light-emitting circuit 11 and the light-receiving circuit 12 are disposed on the same side of the heart rate measurement device 100, the light received by the light-receiving circuit 12 is a light that is reflected back (i.e., a reflected light). If the light-emitting circuit 11 and the light-receiving circuit 12 are disposed on opposing sides of the heart rate measurement device 100, the light received by the light-receiving circuit 12 is a light that is refracted back (i.e., a refracted light).

In order to reduce an interference of an environment to the light emitted by the light-emitting circuit 11 in the heart rate detecting sensor 10 to thereby improve the accuracy of the PPG signal of the blood, the heart rate detecting sensor 10 is preferably disposed at a side of the heart rate measurement device 100 that is in contact with the skin of the user.

Herein, during a non-intense activity, because a relatively smaller portion of a green light is absorbed, thus the green light can be used to accurately detect the PPG signal of the blood during the non-intense activity. During an intense activity, because a relatively smaller portion of a red light is absorbed, thus the red light can be used to accurately detect the PPG signal of the blood during the intense activity.

Herein, two light-emitting sub-circuits are configured in the heart rate measurement device 100, which are respectively configured to emit a green light and a red light, thus the PPG signal of the blood can be accurately detected during different states of the movement (no-intense activity and/or intense activity), thereby ensuring that the heart rate is accurately measured.

In a second aspect, the present disclosure further provides a method for measuring a heart rate of a user by means of the heart rate measurement device according to any of the embodiments as described above. The detailed description of the method can be referenced to the following examples.

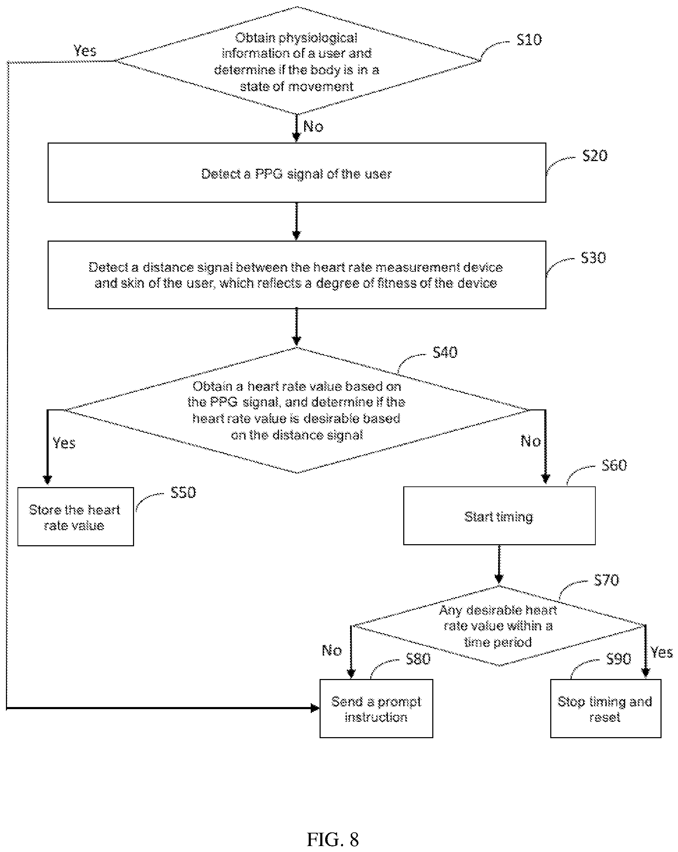

FIG. 8 shows a flow chart of a method for measuring a heart rate of a user by means of a heart rate measurement device according to some embodiments of the present disclosure. The method comprises:
S10: collecting physiological information of the user, determining whether a body of the user is in a state of movement based on the physiological information, and executing S80 if so, or executing S20 if otherwise;

Herein, the physiological information of the user, such as pulse rate, body temperature, and heart beats, is collected by a physiological information collecting portion 60.

S20: detecting a PPG signal of the user;

Herein, the PPG signal of the user can be detected by a sensor employing a PPG heart rate detection.

S30: detecting a distance between the heart rate measurement device 100 and skin of the user, wherein the distance reflects a degree of fitness between the heart rate measurement device 100 and the skin of the user.

Herein, the distance can be detected by a proximity sensor, a distance sensor, or a temperature sensor.

S40: obtaining a heart rate value based on the PPG signal, determining whether the heart rate value is desirable based on the distance, and executing S50 if so, or executing S60 if otherwise;
Herein, the processor 30 determines if the heart rate measurement device 100 is fit with the skin of the user by processing the distance, and further determines if the heart rate values is desirable.

Specifically, in the embodiment employing a temperature sensor for the detection of a distance between the heart rate measurement device 100 and the skin of the user, after receiving an instant temperature detected by the temperature sensor, the processor 30 obtains a temperature change by comparing the instant temperature with a reference temperature.

If the temperature change obtained by the processor 30 is greater than a preset threshold, the heart rate measuring device 100 is not fit with the skin of the user, the heart rate value that has been detected is not desirable, and then S60 is executed.

If the temperature change obtained by the processor 30 is smaller than a preset threshold, the heart rate measuring device 100 is closely fit with the skin of the user, the heart rate value that has been detected is desirable, and then S50 is executed.

Specifically, in the embodiment employing a proximity sensor for the detection of a distance between the heart rate measurement device 100 and the skin of the user, after receiving an instant optical pulse signal detected by the proximity sensor, the processor 30 compares the instant optical pulse signal with a reference optical pulse signal.

If a change of the optical pulse signal is greater than a preset threshold, the heart rate measuring device 100 is not fit with the skin of the user, the heart rate value that has been detected is not desirable, and then S60 is executed.

If the instant optical pulse signal is smaller than the preset threshold, the heart rate measuring device 100 is closely fit with the skin of the user, the heart rate value that has been detected is desirable, and then S50 is executed.

Specifically, in the embodiment employing a distance sensor for the detection of a distance between the heart rate measurement device 100 and the skin of the user, after receiving an instant time interval reflecting a distance between the heart rate measurement device 100 and the skin of the user that is detected by the distance sensor, the processor 30 compares the instant time interval with a preset threshold.

If the instant time interval is greater than a preset threshold, the heart rate measuring device 100 is not fit with the skin of the user, the heart rate value that has been detected is not desirable, and then S60 is executed.

If the instant time interval is smaller than the preset threshold, the heart rate measuring device 100 is closely fit with the skin of the user, the heart rate value that has been detected is desirable, and then S50 is executed.

S50: storing the heart rate value;
Herein the heart rate value can be stored in the memory 40 of the heart rate measurement device, or can be transmitted to an outside terminal through a wireless connection
S60: starting timing;
S70: determining whether there is at least one desirable heart rate value within a time period, and executing S90 if so, or executing S80 if otherwise;
S80: sending a prompt instruction;
S90: stopping timing and resetting.

Herein, the prompt instruction can be delivered via at least one of vibration, light, or sound, and can also be delivered by directly displaying the instant heart rate value. Through these prompt instructions, the user can be prompted to adjust the heart rate measurement device 100 such that it can be fit with the skin of the user.

Herein, there is no limitation to the execution of the heart rate measurement device 100 if none of the heart rate value that has been obtained is desirable. For example, the erroneous heart rate value can be deleted, or can be compensated through a compensation algorithm.

If the prompt instruction is delivered to the user by displaying the prompt instruction, the method can further comprise:
displaying a heart rate value if there is at least one desirable heart rate value.

In the embodiment of the method as described above, by detecting a distance between the heart rate measurement device 100 and skin of a user to thereby examine a degree of fitness between the heart rate measurement device 100 and the skin of the user, it can be determined whether the heart rate value obtained based on the PPG signal is desirable.

If it is determined that the heart rate measurement device 100 is not fit with the skin of the user, the heart rate value is determined as inaccurate and is thus deleted. If it is determined that the heart rate measurement device 100 is fit with the skin of the user, the heart rate value is determined as accurate and is thus stored in the memory 40. As such, the inaccurate heart rate values are deleted and all the heart rate values stored in the memory 40 are accurate, thereby improving the accuracy of measuring the heart rates of the user.

By sending a prompt instruction to a user if a heart rate value that is just obtained is not desirable, the user can be prompted to adjust the heart rate measurement device 100 such that it can be fit with the skin of the user, thereby ensuring that the heart rate values can be accurately obtained, realizing a real-time monitoring of heart rate of the user.

By collecting the physiological information of the user, the heart rate measurement device 100 can thus intelligently recognize a state of movement of the user. If it is determined that the body of the user is in a state of movement, the user is prompted to adjust the heart rate measurement device 100 such that it can be fit with the skin of the user, thereby improving the accuracy of measuring heart rates of the user.

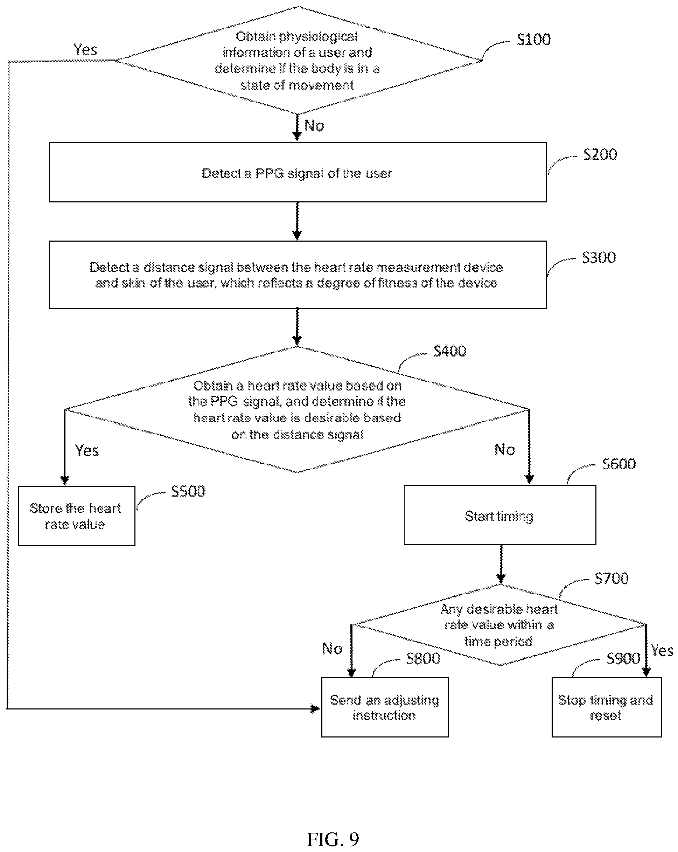

FIG. 9 shows a flow chart of a method for measuring a heart rate of a user by means of a heart rate measurement device according to some other embodiments of the present disclosure. The method comprises:
S100: collecting physiological information of the user, determining whether a body of the user is in a state of movement based on the physiological information, and executing S800 if so, or executing S200 if otherwise;
S200: detecting a PPG signal of the user;
S300: detecting a distance between the heart rate measurement device 100 and skin of the user, wherein the distance reflects a degree of fitness between the heart rate measurement device 100 and the skin of the user.
S400: obtaining a heart rate value based on the PPG signal, determining whether the heart rate value is desirable based on the distance, and executing S500 if so, or executing S600 if otherwise;
S500: storing the heart rate value;
S600: starting timing;
S700: determining whether there is at least one desirable heart rate value within a time period, and executing S900 if so, or executing S800 if otherwise;
S800: sending an adjusting instruction such that an adjusting portion adjusts the heart rate measurement device 100 to be fit with the skin of the user based on the adjusting instruction.
S900: stop timing and resetting.

Herein, the adjusting portion 70 of the heart rate measurement device 100 can receive an adjusting instruction to thereby automatically adjust the heart rate measurement device 100 to be fit with the skin of the user.

By sending an adjusting instruction if a heart rate value that is just obtained is not desirable, the adjusting portion 70 can be configured to automatically adjust the heart rate measurement device 100 to be fit with the skin of the user, thereby increasing the accuracy of measuring heart rates of the user, and further improving the comfortability of wearing.

It can be understood by those of ordinary skill in the art that all or part of the steps in the above mentioned embodiments of the method can be accomplished by means of hardware accompanied with program instructions, which may be stored in a computer-readable storage medium. Executing the program substantially comprises the steps of the method as described above.

Herein, the storage medium includes a variety of media, such as ROM, RAM, disk, or optical disk, which can store codes for the program.

In all of the embodiments of the device and the method as disclosed herein, the PPG-based heart rate determining approach is utilized for the determination of a heart rate of a user. It should be noted that in addition to the PPG-based approach, other approaches, including an ECG-based heart rate determining approach, a pulse oximetry-based heart rate determining approach, or an arterial pressure-based heart rate determining approach, can also be utilized for determining a heart rate of the user. There are no limitations herein.

Although specific embodiments have been described above in detail, the description is merely for purposes of illustration. It should be appreciated, therefore, that many aspects described above are not intended as required or essential elements unless explicitly stated otherwise.

Various modifications of, and equivalent acts corresponding to, the disclosed aspects of the exemplary embodiments, in addition to those described above, can be made by a person of ordinary skill in the art, having the benefit of the present disclosure, without departing from the scope of the disclosure defined in the following claims, the scope of which is to be accorded the broadest interpretation so as to encompass such modifications and equivalent structures.

## Claims

1. A method for monitoring heart rates of a user wearing a heart rate measurement device (100) configured to detect biometric signals of the user, each at a first time interval, the method comprising, each time when a biometric signal is to be detected:
detecting (S20, S30; S200, S300) one biometric signal of the user and a distance signal reflecting and corresponding to a degree of fitness between the heart rate measurement device (100) and skin of the user;
obtaining (S40; S400) a heart rate value based on the one biometric signal, and determining whether the heart rate value is desirable based on the distance signal; and
keeping (S50; S500) the heart rate value if the heart rate value is determined to be desirable, or discarding (S60, S70, S80, S90: S600, S700, S800, S900) the heart rate value if otherwise, the method further comprising, prior to the detecting one biometric signal of the user and a distance signal reflecting and corresponding to a degree of fitness between the heart rate measurement device (100) and skin of the user:
collecting (S10; S 100) physiological information of the user;
determining (S10; S100) whether a body of the user is in a state of movement based on the physiological information; and
sending (S80; S800) an action instruction to the heart rate measurement device (100) to adjust the degree of fitness between the heart rate measurement device (100) and the skin of the user if the body of the user is determined to be in the state of movement.

2. The method of Claim 1, wherein the biometric signals are at least one of photoplethysmography, PPG, signals, electrocardiography, ECG, signals, blood oxygen saturation signals, or arterial pressure signals;
optionally the biometric signals are PPG signals.

3. The method of Claim 1 or 2, wherein in the keeping the heart rate value if the heart rate value is determined to be desirable, or discarding the heart rate value if otherwise, the discarding the heart rate value comprises:
starting timing (S60; S600); and
sending (S80; S800) an action instruction to the heart rate measurement device (100) to adjust the degree of fitness between the heart rate measurement device (100) and the skin of the user if no heart rate value is determined as desirable within a time period (S70; S700), or stopping timing and resetting (S90; S900) if otherwise,
optionally:
the action instruction comprises a prompt instruction configured to prompt the user for manually adjusting the heart rate measurement device (100) to be fit with the skin of the user; and
the sending an action instruction to the heart rate measurement device (100) to adjust the degree of fitness between the heart rate measurement device (100) and the skin of the user comprises:
sending (S80) the prompt instruction to the heart rate measurement device (100),
optionally the action instruction comprises an adjusting instruction configured to instruct the heart rate measurement device (100) for automatically adjusting to be fit with the skin of the user; and
the sending an instruction to the heart rate measurement device (100) to adjust the degree of fitness between the heart rate measurement device (100) and the skin of the user comprises:
sending (S800) the adjusting instruction to the heart rate measurement device (100).

4. The method of Claim 1, wherein:
the action instruction comprises a prompt instruction configured to prompt the user for manually adjusting the heart rate measurement device (100) to be fit with the skin of the user; and
the sending (S80; S800) an action instruction to the heart rate measurement device (100) to manually or automatically adjust the degree of fitness between the heart rate measurement device (100) and the skin of the user comprises:
sending (S80) the prompt instruction to the heart rate measurement device (100),
optionally:
the action instruction comprises an adjusting instruction configured to instruct the heart rate measurement device (100) for automatically adjusting to be fit with the skin of the user; and
the sending (S80; S800) an instruction to the heart rate measurement device (100) to manually or automatically adjust the degree of fitness between the heart rate measurement device (100) and the skin of the user comprises:
sending (S800) the adjusting instruction to the heart rate measurement device (100).

5. The method of any one of Claims 1-4, wherein the distance signal comprises at least one of a temperature signal, an optical pulse signal, or a second time interval signal.

6. The method of Claim 5, wherein the distance signal comprises a temperature signal, wherein:
the detecting (S20, S30; S200, S300) one biometric signal of the user and a distance signal reflecting and corresponding to a degree of fitness between the heart rate measurement device (100) and skin of the user comprises:
detecting an instant temperature of the user by a temperature sensor (20);
and
the obtaining (S40; S400) a heart rate value based on the one biometric signal and determining (S40; S400) whether the heart rate value is desirable based on the distance signal comprises:
obtaining a change of temperature by comparing the instant temperature with a reference temperature; and
determining that the heart rate value is not desirable if the change of temperature is greater than a preset threshold, or determining that the heart rate value is desirable if otherwise,
optionally the distance signal comprises an optical pulse signal, wherein:
the detecting (S20, S30; S200, S300) one biometric signal of the user and a distance signal reflecting and corresponding to a degree of fitness between the heart rate measurement device (100) and skin of the user comprises:
detecting an instant optical pulse signal reflected from the skin of the user by a proximity sensor (20);
and
the obtaining (S40; S400) a heart rate value based on the one biometric signal and determining (S40; S400) whether the heart rate value is desirable based on the distance signal comprises:
obtaining a change of optical pulse signal by comparing the instant optical pulse signal with a reference optical pulse signal; and
determining that the heart rate value is not desirable if the change of optical pulse signal is greater than a preset threshold, or determining that the heart rate value is desirable if otherwise, optionally the distance signal comprises a second time interval signal, wherein:
the detecting (S20, S30; S200, S300) one biometric signal of the user and a distance signal reflecting and corresponding to a degree of fitness between the heart rate measurement device (100) and skin of the user comprises:
detecting an instant second time interval signal of an infrared light reflected from the skin of the user by a distance sensor (20);
and
the obtaining (S40; S400) a heart rate value based on the one biometric signal and determining (S40; S400) whether the heart rate value is desirable based on the distance signal comprises:
comparing the instant second time interval signal with a preset threshold; and
determining that the heart rate value is not desirable if the instant second time interval signal is greater than the preset threshold, or determining that the heart rate value is desirable if otherwise.

7. A heart rate measurement device (100), comprising a heart rate detecting sensor (10), a fitness detecting sensor (20), and a processor (30), wherein:
the heart rate detecting sensor (10) is coupled to the processor (30), and is configured to detect, and to transmit to the processor (30), one biometric signal of a user at a first time interval;
the fitness detecting sensor (20) is coupled to the processor (30), and is configured to detect, and to transmit to the processor (30), a distance signal corresponding to the one biometric signal, wherein the distance signal reflects and corresponds to a degree of fitness between the heart rate measurement device (100) and the skin of the user; and
the processor (30) is configured to obtain a heart rate value based on the one biometric signal, and to determine whether the heart rate value is desirable based on the distance signal,
the heart rate measurement device further comprising a memory (40), coupled to the processor (30) and configured to store the heart rate value if the heart rate value is determined by the processor (30) to be desirable
or to discard the heart rate value if otherwise;
the heart rate measurement device (100) further comprising a physiological information collecting portion (60) and an action portion (50, 70), each coupled to the processor (30), wherein:
prior to the detecting one biometric signal of the user and a distance signal reflecting and corresponding to a degree of fitness between the heart rate measurement device (100) and skin of the user:
the physiological information collecting portion (60) is configured to collect physiological information of the user, and to send the physiological information to the processor (30); and
the processor (30) is further configured to determine whether a body of the user is in a state of movement based on the physiological information, and if so, to send an action instruction to the action portion (50, 70); and
the action portion (50, 70) is configured, upon receiving the action instruction from the processor (30), to act on the heart rate measurement device (100) to adjust the degree of fitness between the heart rate measurement device (100) and the skin of the user.

8. The heart rate measurement device (100) of Claim 7, wherein the processor (30) is further configured to start timing if determining that the heart rate value is not desirable, and to send an action instruction to the action portion (50, 70) if no heart rate value is determined as desirable within a time period, or to stop timing and reset if otherwise.

9. The heart rate measurement device (100) of Claim 8, wherein the action portion (50, 70) comprises a prompting portion (50), and the action instruction comprises a prompt instruction, wherein:
the prompting portion (50) is configured, upon receiving the prompt instruction from the processor (30), to prompt the user for manually adjusting the heart rate measurement device (100) to be fit with the skin of the user,
optionally the action portion (50, 70) comprises an adjusting portion, and the action instruction comprises an adjusting instruction, wherein:
the adjusting portion is configured, upon receiving the adjusting instruction from the processor (30), to automatically adjust the heart rate measurement device (100) to be fit with the skin of the user.

10. The heart rate measurement device (100) of Claim 7, wherein the action portion (50, 70) comprises a prompting portion (50), and the action instruction comprises a prompt instruction, wherein:
the prompting portion (50) is configured, upon receiving the prompt instruction from the processor (30), to prompt the user for manually adjusting the heart rate measurement device (100) to be fit with the skin of the user,
optionally the action portion (50, 70) comprises an adjusting portion (70), and the action instruction comprises an adjusting instruction, wherein:
the adjusting portion (70) is configured, upon receiving the adjusting instruction from the processor (30), to automatically adjust the heart rate measurement device (100) to be fit with the skin of the user,
optionally the adjusting portion (70) comprises a wearing sub-portion (71) and a fastening sub-portion (72), wherein:
the wearing sub-portion (71) is configured to attach the heart rate measurement device (100) on a part of the human body of the user; and
the fastening sub-portion (72) is disposed on the wearing sub-portion (71) and is configured to fasten or loosen the wearing sub-portion (71) to thereby adjust a degree of fitness between the heart rate measurement device (100) and the skin of the user based on the adjusting instruction from the processor (30).

11. The heart rate measurement device (100) of Claim 10, wherein the prompting portion (50) comprises at least one of a display sub-portion, a vibration sub-portion, an audio sub-portion, or a light-emitting sub-portion,
optionally the prompting portion (50) comprises a display sub-portion (50), configured to display the heart rate value.

12. The heart rate measurement device (100) of any one of Claims 7-11, wherein the fitness detecting sensor (20) comprises at least one of a temperature sensor, a proximity sensor, or a distance sensor.

13. The heart rate measurement device (100) of Claim 12, wherein the fitness detecting sensor (20) comprises a temperature sensor (20), wherein:
the temperature sensor (20) is configured to detect an instant temperature of the user; and
the processor (30) is further configured to obtain a change of temperature by comparing the instant temperature with a reference temperature, and to determine that the heart rate value is not desirable if the change of temperature is greater than a preset threshold, or that the heart rate value is desirable if otherwise,
optionally the fitness detecting sensor (20) comprises a proximity sensor (20), wherein:
the proximity sensor (20) is configured to detect an instant optical pulse signal reflected from the skin of the user; and
the processor (30) is further configured to obtain a change of optical pulse signal by comparing the instant optical pulse signal with a reference optical pulse signal, and to determine that the heart rate value is not desirable if the change of optical pulse signal is greater than a preset threshold, or that the heart rate value is desirable if otherwise,
optionally the fitness detecting sensor (20) comprises a distance sensor (20), wherein:
the distance sensor (20) is configured to detect an instant second time interval signal of an infrared light reflected from the skin of the user; and
the processor (30) is further configured to compare the instant second time interval signal with a preset threshold, and to determine that the heart rate value is not desirable if the instant second time interval signal is greater than the preset threshold, or that the heart rate value is desirable if otherwise.

14. A wearable apparatus, comprising a heart rate measurement device (100) according to any one of Claims 7-13, disposed on a side of a user in contact with the skin of the user.

## Patentansprüche

1. Verfahren zum Überwachen von Herzfrequenzen eines Benutzers, der eine Herzfrequenzmessvorrichtung (100) am Körper trägt, die dazu eingerichtet ist, biometrische Signale des Benutzers jeweils in einem ersten Zeitintervall zu detektieren, wobei das Verfahren jedes Mal, wenn ein biometrisches Signal detektiert werden soll, umfasst:
Detektieren (S20, S30; S200, S300) eines biometrischen Signals des Benutzers und eines Distanzsignals, das einen Grad an Passgenauigkeit zwischen der Herzfrequenzmessvorrichtung (100) und der Haut des Benutzers widerspiegelt und diesem entspricht;
Erhalten (S40; S400) eines Herzfrequenzwertes auf der Grundlage des einen biometrischen Signals und Bestimmen, auf der Grundlage des Distanzsignals, ob der Herzfrequenzwert wünschenswert ist; und
Behalten (S50; S500) des Herzfrequenzwertes, falls der Herzfrequenzwert als wünschenswert bestimmt wird, oder Verwerfen (S60, S70, S80, S90; S600, S700, S800, S900) des Herzfrequenzwertes, falls nicht,
wobei das Verfahren des Weiteren vor dem Detektieren eines biometrischen Signals des Benutzers und eines Distanzsignals, das einen Grad an Passgenauigkeit zwischen der Herzfrequenzmessvorrichtung (100) und der Haut des Benutzers widerspiegelt und diesem entspricht, umfasst:
Erfassen (S10; S100) physiologischer Informationen des Benutzers;
Bestimmen (S10; S100), auf der Grundlage der physiologischen Informationen, ob sich ein Körper des Benutzers in einem Zustand der Bewegung befindet; und
Senden (S80; S800) einer Aktionsanweisung an die Herzfrequenzmessvorrichtung (100), um den Grad an Passgenauigkeit zwischen der Herzfrequenzmessvorrichtung (100) und der Haut des Benutzers einzustellen, falls bestimmt wird, dass sich der Körper des Benutzers im Zustand der Bewegung befindet.

2. Verfahren nach Anspruch 1, wobei die biometrischen Signale mindestens eines von Photoplethysmografiesignalen (PPG-Signalen), Elektrokardiografiesignalen (EKG-Signalen), Blutsauerstoffsättigungssignalen oder Arteriendrucksignalen sind;
die biometrischen Signale optional PPG-Signale sind.

3. Verfahren nach Anspruch 1 oder 2, wobei beim Behalten des Herzfrequenzwertes, falls der Herzfrequenzwert als wünschenswert bestimmt wird, oder beim Verwerfen des Herzfrequenzwertes, falls nicht, das Verwerfen des Herzfrequenzwertes umfasst:
Starten einer Zeitmessung (S60; S600); und
Senden (S80; S800) einer Aktionsanweisung an die Herzfrequenzmessvorrichtung (100), um den Grad an Passgenauigkeit zwischen der Herzfrequenzmessvorrichtung (100) und der Haut des Benutzers einzustellen, falls innerhalb eines Zeitraums (S70; S700) kein Herzfrequenzwert als wünschenswert bestimmt wird, oder Stoppen der Zeitmessung und Zurücksetzen (S90; S900), falls doch,
optional:
die Aktionsanweisung eine Aufforderungsanweisung umfasst, die dazu eingerichtet ist, den Benutzer aufzufordern, die Herzfrequenzmessvorrichtung (100) manuell so einzustellen, dass sie an die Haut des Benutzers angepasst ist; und
das Senden einer Aktionsanweisung an die Herzfrequenzmessvorrichtung (100), den Grad an Passgenauigkeit zwischen der Herzfrequenzmessvorrichtung (100) und der Haut des Benutzers einzustellen, umfasst:
Senden (S80) der Aufforderungsanweisung an die Herzfrequenzmessvorrichtung (100),
optional die Aktionsanweisung eine Einstellanweisung umfasst, die dazu eingerichtet ist, die Herzfrequenzmessvorrichtung (100) anzuweisen, sich automatisch so optionnellement en, dass sie an die Haut des Benutzers angepasst ist; und
das Senden einer Anweisung an die Herzfrequenzmessvorrichtung (100), den Grad an Passgenauigkeit zwischen der Herzfrequenzmessvorrichtung (100) und der Haut des Benutzers einzustellen, umfasst: Senden (S800) der Einstellanweisung an die Herzfrequenzmessvorrichtung (100).

4. Verfahren nach Anspruch 1, wobei:
die Aktionsanweisung eine Aufforderungsanweisung umfasst, die dazu eingerichtet ist, den Benutzer aufzufordern, die Herzfrequenzmessvorrichtung (100) manuell so einzustellen, dass sie an die Haut des Benutzers angepasst ist; und
das Senden (S80; S800) einer Aktionsanweisung an die Herzfrequenzmessvorrichtung (100), den Grad an Passgenauigkeit zwischen der Herzfrequenzmessvorrichtung (100) und der Haut des Benutzers manuell oder automatisch einzustellen, umfasst:
Senden (S80) der Aufforderungsanweisung an die Herzfrequenzmessvorrichtung (100),
optional:
die Aktionsanweisung eine Einstellanweisung umfasst, die dazu eingerichtet ist, die Herzfrequenzmessvorrichtung (100) anzuweisen, sich automatisch so einzustellen, dass sie an die Haut des Benutzers angepasst ist; und
das Senden (S80; S800) einer Anweisung an die Herzfrequenzmessvorrichtung (100), den Grad an Passgenauigkeit zwischen der Herzfrequenzmessvorrichtung (100) und der Haut des Benutzers manuell oder automatisch einzustellen, umfasst:
Senden (S800) der Einstellanweisung an die Herzfrequenzmessvorrichtung (100).

5. Verfahren nach einem der Ansprüche 1-4, wobei das Distanzsignal mindestens eines von einem Temperatursignal, einem optischen Impulssignal oder einem Signal eines zweiten Zeitintervalls umfasst.

6. Verfahren nach Anspruch 5, wobei das Distanzsignal ein Temperatursignal umfasst, wobei:
das Detektieren (S20, S30; S200, S300) eines biometrischen Signals des Benutzers und eines Distanzsignals, das einen Grad an Passgenauigkeit zwischen der Herzfrequenzmessvorrichtung (100) und der Haut des Benutzers widerspiegelt und diesem entspricht, umfasst:
Detektieren einer momentanen Temperatur des Benutzers durch einen Temperatursensor (20);
und
das Erhalten (S40; S400) eines Herzfrequenzwertes auf der Grundlage des einen biometrischen Signals und das Bestimmen (S40; S400), auf der Grundlage des Distanzsignals, ob der Herzfrequenzwert wünschenswert ist, umfasst:
Erhalten einer Änderung der Temperatur durch Vergleichen der momentanen Temperatur mit einer Referenztemperatur;
und
Bestimmen, dass der Herzfrequenzwert nicht wünschenswert ist, falls die Änderung der Temperatur größer als eine voreingestellte Schwelle ist, oder Bestimmen, dass der Herzfrequenzwert wünschenswert ist, falls nicht,
optional das Distanzsignal ein optisches Impulssignal umfasst, wobei:
das Detektieren (S20, S30; S200, S300) eines biometrischen Signals des Benutzers und eines Distanzsignals, das einen Grad an Passgenauigkeit zwischen der Herzfrequenzmessvorrichtung (100) und der Haut des Benutzers widerspiegelt und diesem entspricht, umfasst:
Detektieren, durch einen Näherungssensor (20), eines momentanen optischen Impulssignals, das von der Haut des Benutzers reflektiert wird;
und
das Erhalten (S40; S400) eines Herzfrequenzwertes auf der Grundlage des einen biometrischen Signals und das Bestimmen (S40; S400), auf der Grundlage des Distanzsignals, ob der Herzfrequenzwert wünschenswert ist, umfasst:
Erhalten einer Änderung des optischen Impulssignals durch Vergleichen des momentanen optischen Impulssignals mit einem optischen Referenzimpulssignal; und
Bestimmen, dass der Herzfrequenzwert nicht wünschenswert ist, falls die Änderung des optischen Impulssignals größer als eine voreingestellte Schwelle ist, oder Bestimmen, dass der Herzfrequenzwert wünschenswert ist, falls nicht,
optional das Distanzsignal ein Signal eines zweiten Zeitintervalls umfasst, wobei:
das Detektieren (S20, S30; S200, S300) eines biometrischen Signals des Benutzers und eines Distanzsignals, das einen Grad an Passgenauigkeit zwischen der Herzfrequenzmessvorrichtung (100) und der Haut des Benutzers widerspiegelt und diesem entspricht, umfasst:
Detektieren, durch einen Distanzsensor (20), eines momentanen Signals eines zweiten Zeitintervalls eines Infrarotlichts, das von der Haut des Benutzers reflektiert wird;
und
das Erhalten (S40; S400) eines Herzfrequenzwertes auf der Grundlage des einen biometrischen Signals und das Bestimmen (S40; S400), auf der Grundlage des Distanzsignals, ob der Herzfrequenzwert wünschenswert ist, umfasst:
Vergleichen des momentanen Signals eines zweiten Zeitintervalls mit einer voreingestellten Schwelle; und
Bestimmen, dass der Herzfrequenzwert nicht wünschenswert ist, falls das momentane Signal eines zweiten Zeitintervalls größer als die voreingestellte Schwelle ist, oder Bestimmen, dass der Herzfrequenzwert wünschenswert ist, falls nicht.

7. Herzfrequenzmessvorrichtung (100), umfassend einen Herzfrequenzdetektionssensor (10), einen Passgenauigkeitsdetektionssensor (20) und einen Prozessor (30), wobei:
der Herzfrequenzdetektionssensor (10) mit dem Prozessor (30) gekoppelt ist und dazu eingerichtet ist, ein biometrisches Signal eines Benutzers in einem ersten Zeitintervall zu detektieren und an den Prozessor (30) zu senden;
der Passgenauigkeitsdetektionssensor (20) mit dem Prozessor (30) gekoppelt ist und dazu eingerichtet ist, ein dem einen biometrischen Signal entsprechendes Distanzsignal zu detektieren und an den Prozessor (30) zu senden, wobei das Distanzsignal einen Grad an Passgenauigkeit zwischen der Herzfrequenzmessvorrichtung (100) und der Haut des Benutzers widerspiegelt und diesem entspricht; und
der Prozessor (30) dazu eingerichtet ist, auf der Grundlage des einen biometrischen Signals einen Herzfrequenzwert zu erhalten und auf der Grundlage des Distanzsignals zu bestimmen, ob der Herzfrequenzwert wünschenswert ist,
wobei die Herzfrequenzmessvorrichtung des Weiteren einen Speicher (40) umfasst, der mit dem Prozessor (30) gekoppelt und dazu eingerichtet ist, den Herzfrequenzwert zu speichern, falls der Herzfrequenzwert durch den Prozessor (30) als wünschenswert bestimmt wird, oder den Herzfrequenzwert zu verwerfen, falls nicht;
wobei die Herzfrequenzmessvorrichtung (100) des Weiteren einen Abschnitt (60) zum Erfassen physiologischer Informationen und einen Aktionsabschnitt (50, 70) umfasst, die jeweils mit dem Prozessor (30) gekoppelt sind, wobei:
vor dem Detektieren eines biometrischen Signals des Benutzers und eines Distanzsignals, das einen Grad an Passgenauigkeit zwischen der Herzfrequenzmessvorrichtung (100) und der Haut des Benutzers widerspiegelt und diesem entspricht:
der Abschnitt (60) zum Erfassen physiologischer Informationen dazu eingerichtet ist, physiologische Informationen des Benutzers zu erfassen und die physiologischen Informationen an den Prozessor (30) zu senden; und
der Prozessor (30) des Weiteren dazu eingerichtet ist, auf der Grundlage der physiologischen Informationen zu bestimmen, ob sich ein Körper des Benutzers in einem Zustand des Bewegung befindet, und, falls ja, eine Aktionsanweisung an den Aktionsabschnitt (50, 70) zu senden; und
der Aktionsabschnitt (50, 70) dazu eingerichtet ist, bei Empfang des Aktionsbefehls von dem Prozessor (30) auf die Herzfrequenzmessvorrichtung (100) einzuwirken, um den Grad an Passgenauigkeit zwischen der Herzfrequenzmessvorrichtung (100) und der Haut des Benutzers einzustellen.

8. Herzfrequenzmessvorrichtung (100) nach Anspruch 7, wobei der Prozessor (30) des Weiteren dazu eingerichtet ist, eine Zeitmessung zu starten, falls bestimmt wird, dass der Herzfrequenzwert nicht wünschenswert ist, und eine Aktionsanweisung an den Aktionsabschnitt (50, 70) zu senden, falls innerhalb eines Zeitraums kein Herzfrequenzwert als wünschenswert bestimmt wird, oder die Zeitmessung zu stoppen und zurückzusetzen, falls doch.

9. Herzfrequenzmessvorrichtung (100) nach Anspruch 8, wobei der Aktionsabschnitt (50, 70) einen Aufforderungsabschnitt (50) umfasst und die Aktionsanweisung eine Aufforderungsanweisung umfasst, wobei:
der Aufforderungsabschnitt (50) dazu eingerichtet ist, bei Empfang der Aufforderungsanweisung von dem Prozessor (30) den Benutzer aufzufordern, die Herzfrequenzmessvorrichtung (100) manuell so einzustellen, dass sie an die Haut des Benutzers angepasst ist,
optional der Aktionsabschnitt (50, 70) einen Einstellabschnitt umfasst und die Aktionsanweisung eine Einstellanweisung umfasst, wobei:
der Einstellabschnitt dazu eingerichtet ist, bei Empfang der Einstellanweisung von dem Prozessor (30) die Herzfrequenzmessvorrichtung (100) automatisch so einzustellen, dass sie an die Haut des Benutzers angepasst ist.

10. Herzfrequenzmessvorrichtung (100) nach Anspruch 7, wobei der Aktionsabschnitt (50, 70) einen Aufforderungsabschnitt (50) umfasst und die Aktionsanweisung eine Aufforderungsanweisung umfasst, wobei:
der Aufforderungsabschnitt (50) dazu eingerichtet ist, bei Empfang der Aufforderungsanweisung von dem Prozessor (30) den Benutzer aufzufordern, die Herzfrequenzmessvorrichtung (100) manuell so einzustellen, dass sie an die Haut des Benutzers angepasst ist,
optional der Aktionsabschnitt (50, 70) einen Einstellabschnitt (70) umfasst und die Aktionsanweisung eine Einstellanweisung umfasst, wobei:
der Einstellabschnitt (70) dazu eingerichtet ist, bei Empfang der Einstellanweisung von dem Prozessor (30) die Herzfrequenzmessvorrichtung (100) automatisch so einzustellen, dass sie an die Haut des Benutzers angepasst ist,
optional der Einstellabschnitt (70) einen Trage-Teilabschnitt (71) und einen Befestigungs-Teilabschnitt (72) umfasst, wobei:
der Trage-Teilabschnitt (71) dazu eingerichtet ist, die Herzfrequenzmessvorrichtung (100) an einem Teil des menschlichen Körpers des Benutzers anzubringen; und
der Befestigungs-Teilabschnitt (72) an dem Trage-Teilabschnitt (71) angeordnet ist und dazu eingerichtet ist, den Trage-Teilabschnitt (71) zu befestigen oder zu lösen, um dadurch einen Grad an Passgenauigkeit zwischen der Herzfrequenzmessvorrichtung (100) und der Haut des Benutzers auf der Grundlage der Einstellanweisung von dem Prozessor (30) einzustellen.

11. Herzfrequenzmessvorrichtung (100) nach Anspruch 10, wobei der Aufforderungsabschnitt (50) mindestens eines von einem Display-Teilabschnitt, einem Vibrations-Teilabschnitt, einem Audio-Teilabschnitt oder einen Leucht-Teilabschnitt umfasst,
optional der Aufforderungsabschnitt (50) einen Display-Teilabschnitt (50) umfasst, der dazu eingerichtet ist, den Herzfrequenzwert anzuzeigen.

12. Herzfrequenzmessvorrichtung (100) nach einem der Ansprüche 7-11, wobei der Passgenauigkeitsdetektionssensor (20) mindestens einen von einem Temperatursensor, einem Näherungssensor oder einem Distanzsensor umfasst.

13. Herzfrequenzmessvorrichtung (100) nach Anspruch 12, wobei der Passgenauigkeitsdetektionssensor (20) einen Temperatursensor (20) umfasst, wobei:
der Temperatursensor (20) dazu eingerichtet ist, eine momentane Temperatur des Benutzers zu detektieren; und
der Prozessor (30) des Weiteren dazu eingerichtet ist, eine Änderung der Temperatur zu erhalten, indem er die momentane Temperatur mit einer Referenztemperatur vergleicht, und zu bestimmen, dass der Herzfrequenzwert nicht wünschenswert ist, falls die Änderung der Temperatur größer als eine voreingestellte Schwelle ist, oder dass der Herzfrequenzwert wünschenswert ist, falls nicht,
optional der Passgenauigkeitsdetektionssensor (20) einen Näherungssensor (20) umfasst, wobei:
der Näherungssensor (20) dazu eingerichtet ist, ein momentanes optisches Impulssignal zu detektieren, das von der Haut des Benutzers reflektiert wird; und
der Prozessor (30) des Weiteren dazu eingerichtet ist, eine Änderung des optischen Impulssignals zu erhalten, indem er das momentane optische Impulssignal mit einem optischen Referenzimpulssignal vergleicht, und zu bestimmen, dass der Herzfrequenzwert nicht wünschenswert ist, falls die Änderung des optischen Impulssignals größer als eine voreingestellte Schwelle ist, oder dass der Herzfrequenzwert wünschenswert ist, falls nicht, optional der Passgenauigkeitsdetektionssensor (20) einen Distanzsensor (20) umfasst, wobei:
der Distanzsensor (20) dazu eingerichtet ist, ein momentanes Signal eines zweiten Zeitintervalls eines Infrarotlichts zu detektieren, das von der Haut des Benutzers reflektiert wird; und
der Prozessor (30) des Weiteren dazu eingerichtet ist, das momentane Signal eines zweiten Zeitintervalls mit einer voreingestellten Schwelle zu vergleichen und zu bestimmen, dass der Herzfrequenzwert nicht wünschenswert ist, falls das momentane Signal eines zweiten Zeitintervalls größer als die voreingestellte Schwelle ist, oder dass der Herzfrequenzwert wünschenswert ist, falls nicht.

14. Am Körper tragbare Vorrichtung, umfassend eine Herzfrequenzmessvorrichtung (100) nach einem der Ansprüche 7-13, die an einer Seite eines Benutzers in Kontakt mit der Haut des Benutzers angeordnet ist.

## Revendications

1. Procédé pour surveiller la fréquence cardiaque d'un utilisateur portant sur soi un dispositif de mesure de fréquence cardiaque (100) configuré pour détecter des signaux biométriques de l'utilisateur, chacun à un premier intervalle de temps, le procédé comprenant de, chaque fois qu'un signal biométrique doit être détecté :
détecter (S20, S30 ; S200, S300) un signal biométrique de l'utilisateur et un signal de distance reflétant et
correspondant à un degré d'ajustement entre le dispositif de mesure de fréquence cardiaque (100) et la peau de l'utilisateur ;
obtenir (S40 ; S400) une valeur de fréquence cardiaque basée sur le un signal biométrique, et déterminer si la valeur de fréquence cardiaque est souhaitable sur la base du signal de distance ; et
conserver (S50 ; S500) la valeur de fréquence cardiaque s'il est déterminé que la valeur de fréquence cardiaque est souhaitable, ou supprimer (S60, S70, S80, S90 ; S600, S700, S800, S900) la valeur de fréquence cardiaque s'il en était autrement,
le procédé comprenant en outre de, avant la détection du signal biométrique de l'utilisateur et d'un signal de distance reflétant et correspondant à un degré d'ajustement entre le dispositif de mesure de fréquence cardiaque (100) et la peau de l'utilisateur :
collecter (S10 ; S100) des informations physiologiques de l'utilisateur ;
déterminer (S10 ; S100) si un corps de l'utilisateur est dans un état de mouvement sur la base des informations physiologiques ; et
envoyer (S80 ; S800) une instruction d'action au dispositif de mesure de fréquence cardiaque (100) pour régler le degré d'ajustement entre le dispositif de mesure de fréquence cardiaque (100) et la peau de l'utilisateur s'il est déterminé que le corps de l'utilisateur est dans l'état de mouvement.

2. Procédé selon la revendication 1, dans lequel les signaux biométriques sont au moins un parmi des signaux de photopléthysmographie (signaux PPG), des signaux d'électrocardiographie (signaux ECG), des signaux de saturation en oxygène du sang ou des signaux de pression artérielle ;
optionnellement, les signaux biométriques sont des signaux PPG.

3. Procédé selon la revendication 1 ou 2, dans lequel lors de la conservation de la valeur de fréquence cardiaque s'il est déterminé que la valeur de fréquence cardiaque est souhaitable, ou la suppression de la valeur de fréquence cardiaque s'il en était autrement, la suppression de la valeur de fréquence cardiaque comprend de :
lancer un chronométrage (S60 ; S600) ; et
envoyer (S80 ; S800) une instruction d'action au dispositif de mesure de fréquence cardiaque (100) pour régler le degré d'ajustement entre le dispositif de mesure de fréquence cardiaque (100) et la peau de l'utilisateur s'il est déterminé qu'aucune valeur de fréquence cardiaque n'est souhaitable sur une période de temps (S70 ; S700), ou arrêter le chronométrage et réinitialiser (S90 ; S900) s'il en était autrement, optionnellement :
l'instruction d'action comprend une instruction d'invite configurée pour inviter l'utilisateur à régler manuellement l'ajustement du dispositif de mesure de fréquence cardiaque (100) avec la peau de l'utilisateur ; et
l'envoi d'une instruction d'action au dispositif de mesure de fréquence cardiaque (100) pour régler le degré d'ajustement entre le dispositif de mesure de fréquence cardiaque (100) et la peau de l'utilisateur comprend de :
envoyer (S80) l'instruction d'invite au dispositif de mesure de fréquence cardiaque (100), optionnellement, l'instruction d'action comprend une instruction de réglage configurée pour ordonner au dispositif de mesure de fréquence cardiaque (11) de régler automatiquement l'ajustement avec la peau de l'utilisateur ; et
l'envoi d'une instruction au dispositif de mesure de fréquence cardiaque (100) pour régler le degré d'ajustement entre le dispositif de mesure de fréquence cardiaque (100) et la peau de l'utilisateur comprend de : envoyer (S800) l'instruction de réglage au dispositif de mesure de fréquence cardiaque (100).

4. Procédé selon la revendication 1, dans lequel :
l'instruction d'action comprend une instruction d'action configurée pour inviter l'utilisateur à régler manuellement l'ajustement du dispositif de mesure de fréquence cardiaque (100) avec la peau de l'utilisateur ; et
l'envoi (S80 ; S800) d'une instruction d'action au dispositif de mesure de fréquence cardiaque (100) pour ajuster manuellement ou automatiquement le degré d'ajustement entre le dispositif de mesure de fréquence cardiaque (100) et la peau de l'utilisateur comprend de :
envoyer (S80) l'instruction d'invite au dispositif de mesure de fréquence cardiaque (100),
optionnellement :
l'instruction d'action comprend une instruction de réglage configurée pour ordonner au dispositif de mesure de fréquence cardiaque (100) de régler automatiquement l'ajustement avec la peau de l'utilisateur ; et
l'envoi (S80 ; S800) d'une instruction au dispositif de mesure de fréquence cardiaque (100) pour régler manuellement ou automatiquement le degré d'ajustement entre le dispositif de mesure de fréquence cardiaque (100) et la peau de l'utilisateur comprend de :
envoyer (S800) l'instruction de réglage au dispositif de mesure de fréquence cardiaque (100).

5. Procédé selon l'une quelconque des revendications 1 à 4, dans lequel le signal de distance comprend au moins un parmi un signal de température, un signal impulsionnel optique ou un signal de second intervalle de temps.

6. Procédé selon la revendication 5, dans lequel le signal de distance comprend un signal de température, dans lequel :
la détection (S20, S30 ; S200, S300) d'un signal biométrique de l'utilisateur et d'un signal de distance reflétant et correspondant à un degré d'ajustement entre le dispositif de mesure de fréquence cardiaque (100) et la peau de l'utilisateur comprend de :
détecter une température instantanée de l'utilisateur par un capteur de température (20) ; et
l'obtention (S40 ; S400) d'une valeur de fréquence cardiaque basée sur le un signal biométrique et le fait de déterminer (S40 ; S400) si la valeur de fréquence cardiaque est souhaitable sur la base du signal de distance comprend de :
obtenir un changement de température en comparant la température instantanée avec une température de référence ; et
déterminer que la valeur de fréquence cardiaque n'est pas souhaitable si le changement de température est supérieur à un seuil prédéfini, ou déterminer que la valeur de fréquence cardiaque est souhaitable s'il en était autrement,
optionnellement, le signal de distance comprend un signal impulsionnel optique, dans lequel :
la détection (S20, S30 ; S200, S300) d'un signal biométrique de l'utilisateur et d'un signal de distance reflétant et correspondant à un degré d'ajustement entre le dispositif de mesure de fréquence cardiaque (100) et la peau de l'utilisateur comprend de :
détecter un signal impulsionnel optique instantané réfléchi par la peau de l'utilisateur, par un capteur de proximité (20) ;
et
l'obtention (S40 ; S400) d'une valeur de fréquence cardiaque basée sur le un signal biométrique et le fait de déterminer (S40 ; S400) si la valeur de fréquence cardiaque est souhaitable sur la base du signal de distance comprend de :
obtenir un changement de signal impulsionnel optique en comparant le signal impulsionnel optique instantané avec un signal impulsionnel optique de référence ; et
déterminer que la valeur de fréquence cardiaque n'est pas souhaitable si le changement de signal impulsionnel optique est supérieur à un seuil prédéfini, ou déterminer que la valeur de fréquence cardiaque est souhaitable s'il en était autrement,
optionnellement le signal de distance comprend un signal de second intervalle de temps, dans lequel :
la détection (S20, S30 ; S200, S300) d'un signal biométrique de l'utilisateur et d'un signal de distance reflétant et correspondant à un degré d'ajustement entre le dispositif de mesure de fréquence cardiaque (100) et la peau de l'utilisateur comprend de :
détecter un signal instantané de second intervalle de temps d'une lumière infrarouge réfléchie par la peau de l'utilisateur, par un capteur de distance (20) ;
et
l'obtention (S40 ; S400) d'une valeur de fréquence cardiaque basée sur le signal biométrique et le fait de déterminer (S40 ; S400) si la valeur de fréquence cardiaque est souhaitable sur la base du signal de distance comprend de :
comparer le signal de second intervalle de temps instantané avec un seuil prédéfini ; et
déterminer que la valeur de fréquence cardiaque n'est pas souhaitable si le signal de second intervalle de temps instantané est supérieur au seuil prédéfini, ou
déterminer que la valeur de fréquence cardiaque est souhaitable s'il en était autrement.

7. Dispositif de mesure de fréquence cardiaque (100), comprenant un capteur de détection de fréquence cardiaque (10), un capteur de détection d'ajustement (20) et un processeur (30), dans lequel :
le capteur de détection de fréquence cardiaque (10) est couplé au processeur (30) et est configuré pour détecter, et pour transmettre au processeur (30), un signal biométrique d'un utilisateur sur un premier intervalle de temps ;
le capteur de détection d'ajustement (20) est couplé au processeur (30) et est configuré pour détecter, et pour transmettre au processeur (30), un signal de distance correspondant au signal biométrique, dans lequel le signal de distance reflète et correspond à un degré d'ajustement entre le dispositif de mesure de fréquence cardiaque (100) et la peau de l'utilisateur ; et
le processeur (30) est configuré pour obtenir une valeur de fréquence cardiaque basée sur le un signal biométrique, et pour déterminer si la valeur de fréquence cardiaque est souhaitable sur la base du signal de distance,
le dispositif de mesure de fréquence cardiaque comprenant en outre une mémoire (40) couplée au processeur (30) et configurée pour stocker la valeur de fréquence cardiaque si la valeur de fréquence cardiaque est déterminée par le processeur (30) comme étant souhaitable ou pour supprimer la valeur de fréquence cardiaque s'il en était autrement ;
le dispositif de mesure de fréquence cardiaque (100) comprenant en outre une partie de collecte d'informations physiologiques (60) et une partie d'action (50, 70), chacune étant couplée au processeur (30), dans lequel :
avant la détection d'un signal biométrique de l'utilisateur et d'un signal de distance reflétant et correspondant à un degré d'ajustement entre le dispositif de mesure de fréquence cardiaque (100) et la peau de l'utilisateur :
la partie de collecte d'informations physiologiques (60) est configurée pour collecter des informations physiologiques de l'utilisateur, et pour envoyer les informations physiologiques au processeur (30) ; et
le processeur (30) est en outre configuré pour déterminer si un corps de l'utilisateur est dans un état de mouvement sur la base des informations physiologiques, et
si tel est le cas, pour envoyer une instruction d'action à la partie d'action (50, 70) ; et
la partie d'action (50, 70) est configurée pour, à réception de l'instruction d'action du processeur (30), agir sur le dispositif de mesure de fréquence cardiaque (100) pour régler le degré d'ajustement entre le dispositif de mesure de fréquence cardiaque (100) et la peau de l'utilisateur.

8. Dispositif de mesure de fréquence cardiaque (100) selon la revendication 7, dans lequel le processeur (30) est en outre configuré pour lancer un chronométrage s'il est déterminé que la valeur de fréquence cardiaque n'est pas souhaitable, et pour envoyer une instruction d'action à la partie d'action (50, 70) si aucune valeur de fréquence cardiaque n'est déterminée comme étant souhaitable sur une période de temps, ou pour arrêter le chronométrage et le réinitialiser s'il en était autrement.

9. Dispositif de mesure de fréquence cardiaque (100) selon la revendication 8, dans lequel la partie d'action (50, 70) comprend une partie d'invite (50), et l'instruction d'action comprend une instruction d'invite, dans lequel :
la partie d'invite (50) est configurée pour, à réception de l'instruction d'invite du processeur (30), inviter l'utilisateur à régler manuellement l'ajustement du dispositif de mesure de fréquence cardiaque (100) avec la peau de l'utilisateur,
optionnellement, la partie d'action (50, 70) comprend une partie de réglage, et l'instruction d'action comprend une instruction de réglage, dans lequel :
la partie de réglage est configurée pour, à réception de l'instruction de réglage du processeur (30), régler automatiquement l'ajustement du dispositif de mesure de fréquence cardiaque (100) avec la peau de l'utilisateur.

10. Dispositif de mesure de fréquence cardiaque (100) selon la revendication 7, dans lequel la partie d'action (50, 70) comprend une partie d'invite (50), et l'instruction d'action comprend une instruction d'invite, dans lequel :
la partie d'invite (50) est configurée pour, à réception de l'instruction d'invite du processeur (30), inviter l'utilisateur à régler manuellement l'ajustement du dispositif de mesure de fréquence cardiaque (100) avec la peau de l'utilisateur,
optionnellement, la partie d'action (50, 70) comprend une partie de réglage (70), et l'instruction d'action comprend une instruction de réglage, dans lequel :
la partie de réglage (70) est configurée pour, à réception de l'instruction de réglage du processeur (30), régler automatiquement l'ajustement du dispositif de mesure de fréquence cardiaque (100) avec la peau de l'utilisateur,
optionnellement, la partie de réglage (70) comprend une sous-partie de port (71) et une sous-partie de fixation (72), dans lequel :
la sous-partie de port (71) est configurée pour fixer le dispositif de mesure de fréquence cardiaque (100) sur une partie du corps humain de l'utilisateur ; et
la sous-partie de fixation (72) est disposée sur la sous-partie de port (71) et est configurée pour fixer ou desserrer la sous-partie de port (71) pour ainsi régler un degré d'ajustement entre le dispositif de mesure de fréquence cardiaque (100) et la peau de l'utilisateur sur la base de l'instruction de réglage du processeur (30).

11. Dispositif de mesure de fréquence cardiaque (100) selon la revendication 10, dans lequel la partie d'invite (50) comprend au moins un parmi une sous-partie d'affichage, une sous-partie de vibration, une sous-partie audio ou une sous-partie d'émission de lumière,
optionnellement, la partie d'invite (50) comprend une sous-partie d'affichage (50), configurée pour afficher la valeur de fréquence cardiaque.

12. Dispositif de mesure de fréquence cardiaque (100) selon l'une quelconque des revendications 7 à 11, dans lequel le capteur de détection d'ajustement (20) comprend au moins un parmi un capteur de température, un capteur de proximité ou un capteur de distance.

13. Dispositif de mesure de fréquence cardiaque (100) selon la revendication 12, dans lequel le capteur de détection d'ajustement (20) comprend un capteur de température (20), dans lequel :
le capteur de température (20) est configuré pour détecter une température instantanée de l'utilisateur ; et
le processeur (30) est en outre configuré pour obtenir un changement de température en comparant la température instantanée avec une température de référence, et pour déterminer que la valeur de fréquence cardiaque n'est pas souhaitable si le changement de température est supérieur à un seuil prédéfini, ou que la valeur de fréquence cardiaque est souhaitable s'il en était autrement, optionnellement, le capteur de détection d'ajustement (20) comprend un capteur de proximité (20), dans lequel :
le capteur de proximité (20) est configuré pour détecter un signal impulsionnel optique instantané réfléchi par la peau de l'utilisateur ; et
le processeur (30) est en outre configuré pour obtenir un changement de signal impulsionnel optique en comparant le signal impulsionnel optique instantané avec un signal impulsionnel optique de référence, et pour déterminer que la valeur de fréquence cardiaque n'est pas souhaitable si le changement du signal impulsionnel optique est supérieur à un seuil prédéterminé, ou que la valeur de fréquence cardiaque est souhaitable s'il en était autrement, optionnellement, le capteur de détection d'ajustement (20) comprend un capteur de distance (20), dans lequel :
le capteur de distance (20) est configuré pour détecter un signal instantané de second intervalle de temps d'une lumière infrarouge réfléchie par la peau de l'utilisateur ; et
le processeur (30) est en outre configuré pour comparer le signal instantané de second intervalle de temps avec un seuil prédéfini, et pour déterminer que la valeur de fréquence cardiaque n'est pas souhaitable si le signal instantané de second intervalle de temps est supérieur au seuil prédéfini, ou que la valeur de fréquence cardiaque est souhaitable s'il en était autrement.

14. Appareil à porter sur soi, comprenant un dispositif de mesure de fréquence cardiaque (100) selon l'une quelconque des revendications 7 à 13, disposé sur un côté d'un utilisateur en contact avec la peau de l'utilisateur.
